# EUROPEAN PATENT APPLICATION

(11) **EP 4 549 551 A1**
(43) Date of publication of application: **07.05.2025**
(21) Application number: 23207196.9
(22) Date of filing: 31.10.2023
(51) Int. Cl.: C12N 5/071

(54) **THE METHOD FOR THE GENERATION OF IMMORTALIZED INTESTINAL EPITHELIAL CELLS**

(71) Applicant: Vilnius University, 01513 Vilnius (LT)
(72) Inventor: Krestnikova, Natalija, Vilnius (LT); Baltriukiene, Daiva, Vilnius (LT); Bukelskiene, Virginija, Vilnius (LT); Baltrukonyte, Emilija, Vilnius (LT); Iesmantaite, Monika, Vilnius (LT)
(74) Representative: AAA Law

(57) **Abstract**

The present invention provides a novel method for obtaining a homogenous population of mammalian colon epithelial crypts, as well as a homogenous population of intestinal epithelial cells. The present invention provides a novel method for immortalization of primary colon epithelial cells by inactivation of the CDKN2A locus and expression of human telomerase reverse transcriptase (hTERT) transgene. This method can be used for digestive system fundamental research, disease modelling and drug screening.

## Description

### FIELD OF INVENTION

The present invention relates to a method for generation of homogenous population of mammalian colon epithelial crypts, as well as intestinal epithelial cells, in particular, mouse colon epithelial crypts and cells are concerned. It further relates to a method of obtaining immortalized intestinal epithelial cells.

### BACKGROUND OF INVENTION

In the last three decades, a rapid increase in intestinal diseases such as cancer, inflammatory bowel disease (IBD) or food allergies has been observed in industrialized countries. Accordingly, there is a need for a better understanding of the cellular and molecular mechanisms that control intestinal epithelial cell (IEC) functions in health and disease (Wilson et al., 2021).

The intestinal epithelium forms the largest mucosal surface in the body with a single layer of polarized cells covering a surface area of -400 m². The intestinal mucosa consists of luminal projections called villi and invaginations called crypts. The villi and the surface of the colon crypts are covered with differentiated epithelial cells, while the base of the crypts consists of stem cells that ensure rapid renewal of the intestinal epithelium, which has a turnover from 3 to 7 days. Intestinal stem cells (ISCs) are important not only for maintaining gut homeostasis, but also for repairing damaged epithelium. The intestinal epithelium not only plays a role in digestion and water/nutrient absorption, but also acts as a physical and biochemical barrier separating host tissues from microorganisms and digestive end products in the intestinal lumen. The latter properties of the intestinal epithelium are a key factor in the maintenance of intestinal homeostasis, interaction with pathogens and mucosal inflammation (Wilson et al., 2021; Koo et a., 2011).

A major limitation in basic and applied research on the biology of the intestinal epithelium is the availability of physiologically relevant in vitro models. The most common models are based on the use of epithelial cell lines derived from tumours. These cells can be easily maintained and genetically modified, but often show significant structural or functional alterations as compared to their in vivo counterparts (EP2675894). Therefore, primary cells better reflect the situation in vivo. However, their short survival in 2D culture precludes their use in functional in vitro studies. Stable intestinal epithelial cell lines were also obtained from transgenic mice carrying the SV40 large T antigen. Although these cell lines have many characteristics of IECs, this approach cannot be used to develop cell lines with similar characteristics from other mouse strains such as gene-deficient or transgenic mice (Matano et al., 2015; Andersson-Rolfa et al., 2016).

Until recently, long-term in vitro culture of normal intestinal stem cells was only possible using three-dimensional (3D) organoids (Hua Zhao et al., 2022). Organoids are spherical epithelial tissue structures, usually smaller than 1 mm, cultured in 3D hydrogels with media that mimic ISC niche signalling. The ISCs in organoids differentiate stochastically or by induction with native factors or small molecules, providing a very accurate model of the gut epithelium. However, the utility of 3D organoids is limited by complex 3D culture methods and challenging imaging and quantification through the Z-planes of 3D hydrogels. In addition, the intestinal epithelium in vivo consists of a layer of cells that carry out region-specific physiological processes. EP3901250 provides a method for producing an intestinal organoid derived from a pluripotent stem cell, wherein the function of an intestinal organoid was enhanced by adding a predetermined low-molecular weight compound. In addition, a two-dimensional evaluation model, which is more suitable for high-throughput assay, was provided. However, such method is time and material consuming and computationally expensive due to the need to obtain and differentiate of endoderm-like cell into an intestinal stem cells. Two-dimensional cultures of the intestinal epithelium can be generated using intestinal stem cells derived from primary tissue sources. These 2D cultures are emerging as an attractive and versatile alternative to 3D organoid cultures. For example, in EP2675894, a coculture model, based on Caco-2 absorptive cells and HT29 goblet cells to test the transport of active substances and nutrients in the intestine, was developed. Such model is a 2D model of the intestine consisting of well-developed polarized monolayers of intestinal cells joined by tight intercellular junctions that restrict the passage of drug molecules and ions. However, this model is based on the use of cells of tumour origin.

No transgenesis and gene editing approaches have yet been developed for intestinal stem cells grown in 2D monolayers. Therefore, the development of new, easily obtainable and reproducible 2D colon cell models of genetically engineered epithelial cells is highly desirable since intestinal epithelium plays a crucial role in mucosal immunology and host microbial homeostasis. In particular, there is a need for highly reproducible and robust two-dimensional (2D) in vitro intestinal models with simultaneous apical and basolateral access. Establishing intestinal cell lines capable of self-renewal and long-term growth as monolayers could therefore solve many of the challenges of 3D organoid cultures and serve as a valuable model for studying intestinal stem cell biology, disease modelling and drug screening. The presently disclosed invention provides solution to these and other problems.

### SUMMARY OF INVENTION

The present invention provides a novel method for obtaining a homogenous population of colon epithelial crypts, as well as a homogenous population of intestinal epithelial cells. The epithelial cells isolated by the present method are not contaminated by cells from the underlying muscle and mesenchymal tissues. The present invention further provides a novel method for immortalization of primary colon epithelial cells by inactivation of the CDKN2A locus and expression of human telomerase reverse transcriptase (hTERT) transgene. In contrast to the available epithelial cell 2D models, the present invention is based on the use of normal immortalized intestinal epithelial cells that can grow long-term *in vitro.*

In particular, a method for isolating colon epithelial crypts is disclosed, comprising the steps: a) providing a dissected colon; b) opening a dissected colon longitudinally; c) incubating the colon in a non-enzymatic cell recovery solution; d) shaking the colon in sucrose solution; e) subjecting the crypts to centrifugation, and f) removing supernatant, thereby obtaining isolated epithelial crypts. Further, a method for obtaining primary intestinal epithelial cells is disclosed, comprising: seeding isolated intestinal epithelial crypts in a culture medium comprising a Rho-kinase inhibitor, and subjecting the culture medium to the conditions suitable for cultivating epithelial cells. Preferably, the intestinal epithelial crypts are isolated by the above-mentioned method for isolating colon epithelial crypts.

Yet further, a method for preparation of immortalized intestinal epithelial cells from primary intestinal epithelial cells is disclosed, comprising the steps: providing primary intestinal epithelial cells; immortalizing the cells by: (i) Inactivating the CDKN2A locus, and (ii) introducing an hTERT transgene.

Further, isolated colon epithelial crypts, isolated intestinal epithelial cells, and immortalized intestinal epithelial cells obtained by these methods are disclosed.

### BRIEF DESCRIPTION OF DRAWINGS

Embodiments of the present invention will now be described with reference to the accompanying drawings, which are provided with corresponding reference numerals and in which:

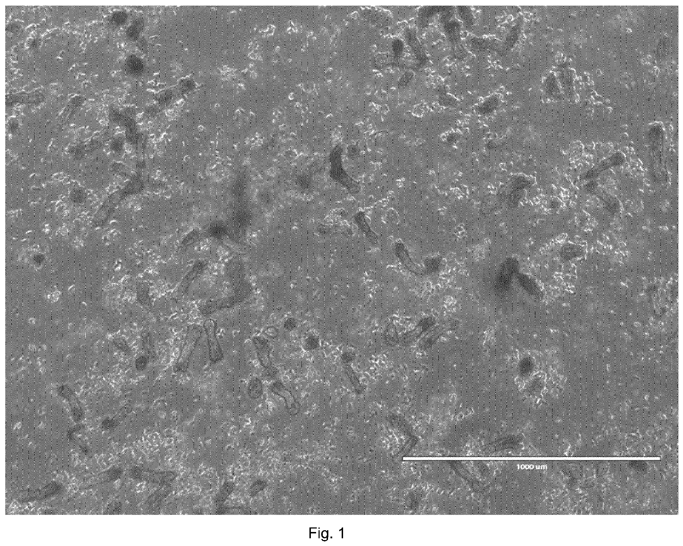

Figure 1 shows isolated colon epithelial crypts under inverted phase-contrast microscope. The image depicts a field of a 30 µl of crypt suspension under the bright field microscope. Intact/undamaged crypts consisting of multiple epithelial cells can be seen. After appropriate dilution, crypts are plated on Rat Tail Collagen (Gibco) precoated dishes in order to obtain monolayer of colon epithelial cells. Scale bar 1000 µm.

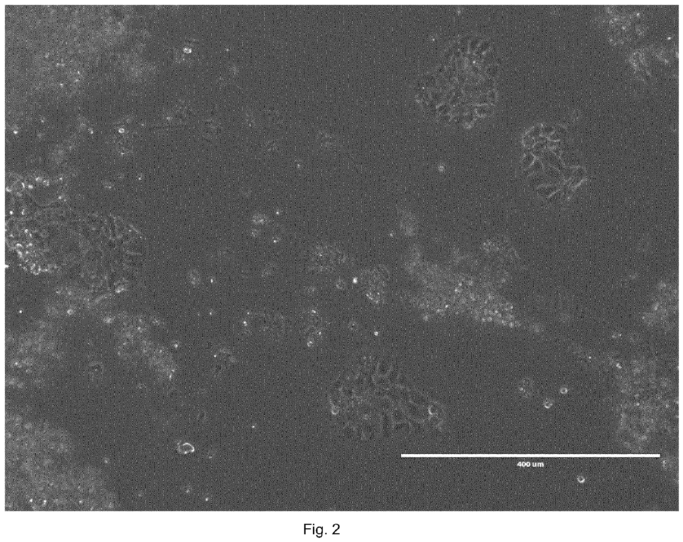

Figure 2 shows murine colon epithelial culture progression under the inverted phase-contrast microscope 24 hours after isolation from the crypts. Multiple colonies of attached intestinal epithelial cells can be seen. The colonies display characteristics of epithelial cells such as paving-stone-like arrangement and a tightly packed pattern. Each cell has polygonal, flattened shape with a large, oval nucleus, typical features of epithelial cells. Other cell types that are not epithelial origin are not observed in culture. Scale bar 400 µM.

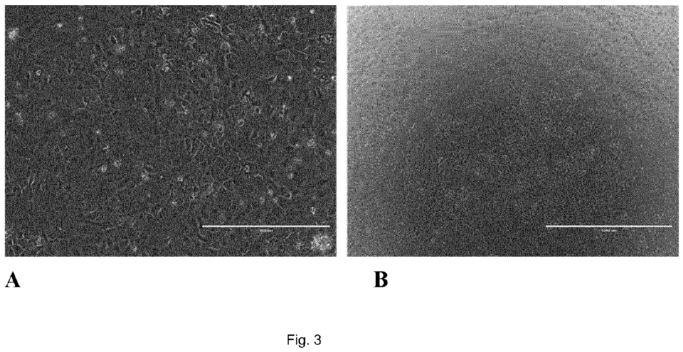

Figure 3 shows colon epithelial cell culture progression under the inverted phase-contrast microscope following 72 h of culturing. Cells continued to grow until confluence was reached. Scale bar 400 and 1000 µM.

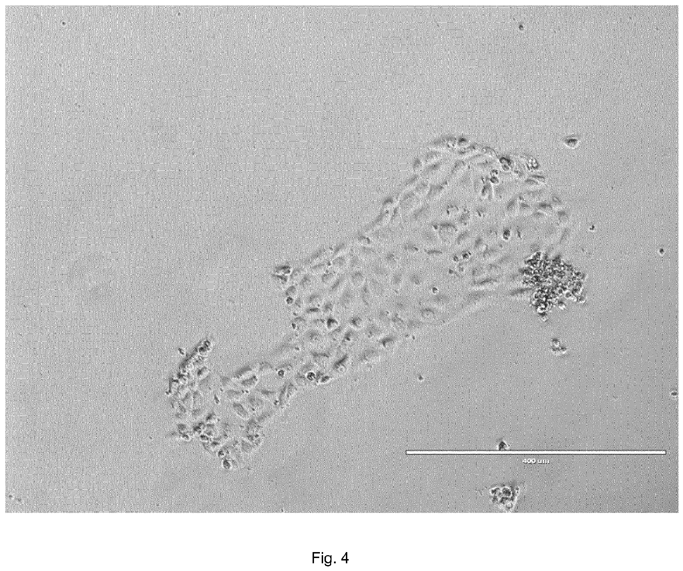

Figure 4 shows culture of immortalized colon epithelial cells with polarized tight monolayer typical for normal intestinal epithelial cells. No morphological changes have been observed. Scale bar 400 µm.

### DETAILED DESCRIPTION OF THE INVENTION

### Isolation of colon epithelial crypts

Currently available protocols for separating adult intestinal epithelial cells from the underlying muscle and mesenchymal tissues typically require long incubation times, harsh mechanical treatment and exposure to proteases. The drawbacks of these methods include crypt fragmentation, contamination with other cell types and reduced viability of the isolated epithelial cells (Ali Moussavi et al., 2018). For example, according to the protocol provided by StemCell Technologies, when isolating the intestinal epithelial cell from mice, it is recommended to use a colon from two or three mice to ensure greater seeding density when performing the protocol for the first time. The intestine is cut into segments up to 2 mm long and then rinsed several times rinsing with PBS. After incubation in Cell Dissociation Reagent, the collected tissue segments are resuspended in PBS and settled by gravity. The supernatant obtained is filtered through a 70 µm filter and the filtrate is collected. The filter is discarded and the filtration step is repeated in order to generate up to 4 filtrate fractions. The fractions obtained are further examined under a bright field microscope. If the fractions have a high proportion of thin fibrous material, the filtration steps are repeated to generate fractions 5 and 6. The fractions are resuspended separately and centrifuged, leaving the pellet in each tube for further processing.

In contrast to the described protocol that is established in the art, the isolation method provided by the present invention is easy to perform even for the first time and is less time- and material-consuming as multiple filtration and generation of filtrate fractions are not required to obtain a pure fraction of colon crypts free of cells of non-epithelial origin. In the method of the present invention, the colon is not cut into small pieces, but instead it is sufficient to open a dissected colon longitudinally and proceed further with the isolation method. Furthermore, unlike the example protocols established in the art, the combination of chemical and mechanical forces in the hereby disclosed isolation method ensures a good yield of crypts even from a lower amount of colon, for example from one mouse colon.

In another exemplary protocol established in the art (Ren et al., 2011), the method of isolation of mouse intestinal cells includes mincing of embryonic intestine into 1 mm long fragments followed by incubation in type I collagenase and hyaluronidase solution. After incubation step, the isolated crypts are centrifuged and washed, and plated out on cultivation dishes. However, this method, while it consists of fewer isolation steps, requires additional steps for purification of obtained intestinal epithelial cell cultures. As mentioned, the fibroblasts were usually mixed with intestinal epithelial cells that grow either in groups or scattered. The fibroblasts were detached from the culture by digestion with trypsin, as they have different tolerance to trypsin exposure. The fibroblasts were then washed off and the IECs were left in the flask.

In contrast to the protocol by Ren et al., the method of the present invention does not rely on the use of proteolytic enzymes. Instead, a non-enzymatic cell recovery solution that depolymerizes the extracellular matrix of the basement membrane is used. As a result, the obtained preparations of epithelial crypts are free from contamination by other cell types. Therefore, no additional purification step with a proteolytic enzyme trypsin is required for epithelial cell cultures prepared from crypts.

The new method of isolation as described by the present disclosure is adapted to provide intestinal crypts free of contamination of other cell types. According to the present method, preincubation of colon in nonenzymatic Cell Recovery Solution (optionally followed by incubation in EDTA solution), enables gentle separation of crypts, of the colon epithelial crypts by applying mechanical forces, e.g. shaking in sucrose solution. The result is intact /undamaged epithelial crypts free of cells of non-epithelial origin (i.e. are not contaminated by cells from the underlying muscle and mesenchymal tissues). Figure 1 shows intact crypts of the mouse intestine after the isolation procedure. As a result, a researcher is provided with ideal material for generating primary cultures of intestinal epithelial cells.

The process for crypt extraction with minimal generation of tissue debris and cells from the subepithelial layer is explained below in detail. The proposed method can be easily used for efficient establishment of primary mouse colon epithelial cells growing as monolayers. The provided method can also be efficiently used for establishment of primary epithelial cultures from different parts of intestine, e.g. small intestine.

In a first aspect of the present disclosure, a method for isolating colon epithelial crypts is disclosed, the method comprising the below listed steps:
a) providing a dissected colon;
b) opening a dissected colon longitudinally;
c) incubating the colon in a non-enzymatic cell recovery solution;
d) shaking the colon in sucrose solution;
e) subjecting the crypts to centrifugation, and
f) removing supernatant, thereby obtaining isolated epithelial crypts.

Non-enzymatic cell recovery solutions are well known in the art and will be familiar to the skilled person. The cell recovery solution acts to breakdown the extracellular support matrix to release the organoids without damage. Suitable cell recovery solutions include, for example, Corning Cell Recovery Solution.

In second aspect, the method for isolating colon epithelial crypts may further comprise, after step c) and before step d), a step of incubating the colon in a chelating solution. In some embodiments, the chelating solution is EDTA or EGTA solution. In further embodiments, the chelating solution is a 50 mM EDTA or EGTA solution.

In a third aspect, the method for isolating colon epithelial crypts may further comprise a step of seeding the isolated crypts in the culture medium.

In the presently disclosed method the Rho-kinase inhibitor (for example, Y-27632) may be used, for example, during the centrifugation of the crypts and/or during the subsequent seeding of the crypts onto culture dishes containing culture medium. This avoids lysis of some cells due to the high sensitivity of epithelial cells to anoikis and results in higher number of viable cells. Preferably, the Rho-kinase inhibitor is used at a concentration of 10 µM.

In a fourth aspect, in the method for isolating colon epithelial crypts, the step e) and/or step g) is performed in the presence of a Rho-kinase inhibitor. Rho-kinase inhibitor, also known as Rock inhibitor, refers to any compound which acts to inhibit the activity of rho-protein kinase. Rho-kinase inhibitor useful in the hereby disclosed method may be Y-27632, which inhibits both ROCK1 (Ki = 220 nM) and ROCK2 (Ki = 300 nM) by competing with ATP for binding to the catalytic site. Other Rho-kinase inhibitors with the same mechanisms of action could be used as substituents.

The method for isolating colon epithelial crypts as described herein may be used for isolation of colon epithelial crypts from a mammal colon. In some embodiments, the crypts are obtained from a rodent colon. In preferred embodiments, the crypts are obtained from a mouse colon. In some examples, the crypts isolated according to the presently disclosed method are obtained from normal mice without genetic mutation. In some other examples, the crypts isolated according to the presently disclosed method are obtained from transgenic mice with a known genetic mutation. The pure fraction of intestinal epithelial cells derived from transgenic mice can be used to produce a disease model.

Further, a method for obtaining primary intestinal epithelial cells is provided, comprising: seeding isolated intestinal epithelial crypts in a culture medium comprising a Rho-kinase inhibitor; and subjecting the culture medium to the conditions suitable for cultivating epithelial cells. The cells are cultivated in a monolayer. Preferably, the intestinal epithelial crypts are obtained by the method for isolating colon epithelial crypts as described in the present disclosure.

### Immortalization of colon epithelial cells

Another aspect of the invention concerns a method for colon epithelial cells immortalization utilizing inactivation of the CDKN2A locus concomitantly with ectopic expression of an hTERT transgene. The proposed immortalization method allows cells to evade senescence in the absence of niche-specific growth factors and continue to divide.

The present invention provides a strategy for rapid attenuation of senescence signals simultaneously with the expression of hTERT to prevent spontaneous alterations that are dispensable for immortalization via CRISPR-mediated inactivation of the CDKN2A locus. This method facilitates direct immortalization of colon epithelial cells without dependence on the spontaneous silencing of p16INK4A expression, which may not be able to stably maintain a low expression under all growth conditions.

The advantage of the proposed method is that immortal cells thus obtained retain the characteristics of normal cells including normal p53 and pRB pathways and near normal karyotypes.

The immortalization procedure may be carried out on cells not older than fourth passage cells. Besides, cell immortalization may be carried out on the second day after isolation and seeding of crypts on an actively proliferating culture.

Moreover, the disclosed immortalization method creates a colon epithelial cell model consisting of cytogenetically normal and nontumorigenic intestinal cells. Furthermore, the proposed method can be used for generation of immortalized epithelial cells from the small intestine.

Another advantage of the proposed method is that thus obtained renewable colon epithelial model is characterized by a polarized monolayer grow resembling naturally occurring intestinal epithelium. Advantageously, the intestinal epithelial cell models of intestine according to the present invention may be used for small, medium and/or high throughput drug-screening and for validation (or invalidation) of drug candidates for digestive system-related diseases.

In some aspects, a method for preparation of immortalized intestinal epithelial cells from primary intestinal epithelial cells is disclosed herein, comprising the steps:
a) Providing primary intestinal epithelial cells;
b) Immortalizing the cells by:
   (i) Inactivating the CDKN2A locus, and
   (ii) introducing an hTERT transgene.

In some embodiments, the steps (i) and (ii) are performed sequentially. In other embodiments, the steps (i) and (ii) are performed simultaneously.

In some aspects, in the method for preparation of immortalized intestinal epithelial cells the primary intestinal epithelial cells are obtained by the method for isolating colon epithelial crypts and a method for obtaining primary intestinal epithelial cells as described by the present disclosure.

In some aspects, an immortalized epithelial cell is disclosed, comprising: an inactivated CDKN2A locus, and expressing hTERT transgene.

### Definitions

Unless defined otherwise, all technical and scientific terms used herein have the meaning commonly understood by a person skilled in the art to which this invention belongs.

The following terms have got the below indicated meanings, unless specified otherwise:
IBD - inflammatory bowel diseases; IEC - intestinal epithelial cell; ISC- intestinal stem cells; 3D - three-dimensional; 2D - two-dimensional; CDKN2A - cyclin dependent kinase inhibitor 2A; hTERT - human telomerase reverse transcriptase.

The term "immortalized cell" refers in general to primary cells whose telomeres and/or tumour suppressor genes have been altered.

The term "medium" also refers to as cell culture medium or culture medium, refers to a medium for culturing cells containing nutrients that maintain cell viability and support cell proliferation. 3dGROTM L-WRN Conditioned Media Supplement manufactured by Sigma Aldrich is a formulation based on DMEM/F-12 media containing the 3 growth factors Wnt3a (W), R-spondin-3 (R), and Noggin (N). The term "L-WRN cultivation media" refers to the advanced DMEM/F12 media (Gibco) combined with 3dGROTM L-WRN Conditioned Media Supplement at 1:1 ratio, supplemented with 100x commercially available glutamine, 15 mM HEPES, 2 mM N-acetyl-L-cysteine, N2 supplement, B27 supplement, 200 ng/ml EGF and 100x commercially available antibiotics.

### EXAMPLES

The following is information on specific examples describing implementation of the invention. Embodiments of the invention are provided for illustration; they do not limit the scope of the invention.

### Example 1. Method for isolation of colon epithelial crypts and obtaining of colon epithelial cell monolayers

The procedure by which colon crypts were isolated is described as follows:
1. Euthanize 8-12 weeks old C57BI/6 mice using an approved method of euthanasia. Remove colon using sterile dissection scissors and forceps. Place dissected colon into ice-cold PBS without Ca²⁺ and Mg2+. Using ice cold PBS without Ca²⁺ and Mg²⁺ flash off the feces with 10 ml syringe, fitted with a 20 G feeding tube.
2. Cut the colon longitudinally and wash it twice in ice-cold PBS without Ca²⁺ and Mg²⁺.
3. Place the colon into a 15 ml tube with ice-cold Cell Recovery Solution (Corning) for 30 min.
4. Discard the Cell Recovery Solution and add 10 ml of 50 mM EDTA solution in PBS. Rotate for 30 min at 4C.
5. Replace EDTA with 5 ml of sucrose (43 mM in PBS without Ca²⁺ and Mg²⁺). Shake it in a vertical position (by hand or in a shaker), decant the sucrose solution into a new 15 ml tube and repeat the shaking step for a total of 10 ml of crypts in shaking buffer.
6. Centrifuge the tube with isolated crypts at 400 x g for 5 min at 4C.
7. Remove supernatant and resuspend crypts in L-WRN cultivation media supplemented with Rho-kinase inhibitor.
8. Count the number of crypts in the obtained suspension. Plate the crypts on culture dishes precoated with Rat Tail Collagen Solution (5 ug/cm²) at a concentration of 500-1000 crypts/cm². Incubate in a 5% CO2 incubator at 37C.
9. The following day aspirate the media with unattached cells and add cultivation media without Rho-kinase inhibitor.

### Example 2. Passaging of colon epithelial cells

1. Replace the cultivation media with fresh cultivation media every 2 or 3 days until the cells reach 80 % confluency. Cells can be passaged at a ratio of 1:4 in the presence of Rho-kinase inhibitor.
2. To passage cells, aspirate cultivation media and 2x wash cells with PBS. Add Accutase solution (Gibco) and place the plate in a 37°C, 5% CO² humid incubator for 5-10 minutes to detach cells from the plate surface.
3. Transfer Accutase with detached cells to conical tube.
4. Centrifuge the conical tube at 300 x g at 4°C for 5 minutes.
5. Aspirate carefully the supernatant and resuspend cell pellet in the appropriate volume of cultivation media. Plate cells on collagen precoated cultivation dish. Rho-kinase inhibitor may be added during every cell passage.
Established cultures of intestinal epithelial cells usually reach high confluency within 72 hours and should be passaged every 3-4 days. The Rho-kinase inhibitor should be added each time the cells are passaged.

### Example 3. Immortalization of cells

We used a genetic approach, the Clustered Regularly Interspaced Short Palindromic Repeats (CRISPR/CRISPR-associated (Cas) system (CRISPR/Cas) to introduce CDKN2a INK4a/Arf inactivation. LentiCRISPRv2-sgCDKN2A vector was generated using sgCDKN2a (gcgctgcgtcgtgcaccggg) targeting exon 3 of the CDKN2a (cccggtgcacgacgcagcgc).

Cells were co-transduced with lentiviral pLV-hTERT-IRES-hygro (Addgene#85140) and lentiCRISPRv2-sgCDKN2A and selected with 25 µg/mL hygromycin and 0.25 µg/mL puromycin.

### References

Ali Moussavi Nik & Peter Carlsson. Separation of intact intestinal epithelium from mesenchyme. BIOTECHNIQUES, 2018. https://doi.org/10.2144/000114055
Hui Jun Ren, Jing Cui, Zhong Quan Wang, Ruo Dan Liu. Normal Mouse Intestinal Epithelial Cells as a Model for the in vitro Invasion of Trichinella spiralis Infective Larvae. PloS ONE, 2011. doi:10.1371 /journal.pone.0027010
Sarah S. Wilson, Martha Mayo, Terry Melim, Heather Knight, Lori Patnaude, Xiaoming Wu, Lucy Phillips, Susan Westmoreland, Robert Dunstan, Edda Fiebiger, Sonia Terrillon. Optimized Culture Conditions for Improved Growth and Functional Differentiation of Mouse and Human Colon Organoids. Front. Immunol, 2021. https://doi.org/10.3389/fimmu.2020.547102
Mami Matano, Shoichi Date, Mariko Shimokawa, Ai Takano, Masayuki Fujii, Yuki Ohta, Toshiaki Watanabe, Takanori Kanai, Toshiro Sato. Modeling colorectal cancer using CRISPR-Cas9-mediated engineering of human intestinal organoids. Nature medicine, 2015. doi:10.1038/nm.3802
Bon-Kyoung Koo, Daniel E Stange, Toshiro Sato, Wouter Karthaus, Henner F Farin, Meritxell Huch, Johan H van Es, Hans Clevers. Controlled gene expression in primary Lgr5 organoid cultures. Nature methods, 2011, 9(1):81-3. doi:10.1038/nmeth.1802
Amanda Andersson-Rolfa, Alessandra Merendaa, Roxana C, Taibo Lia, Sabine Dietmanna, Bon-Kyoung Koo. Simultaneous paralogue knockout using a CRISPR-concatemer in mousecrossmark small intestinal organoids. Developmental Biology 420 (2016) 271-277.http://dx.doi.org/10.1016/j.ydbio.2016.10.016
Hua Zhao, Yulan Cheng, Andrew Kalra, Ke Ma, Yueyuan Zheng, Benjamin Ziman, Caitlin Tressler, Kristine Glunde, Eun Ji Shin, Saowanee Ngamruengphong, Mouen Khashab,Vikesh Singh, Robert, Anders, Simran Jit, Nicolas Wyhs, Wei Chen, Xu Li1, De-Chen Lin1, Stephen J. Meltzer. Generation and multiomic profiling of a TP53/CDKN2A double-knockout gastroesophageal junction organoid model. SCIENCE TRANSLATIONAL MEDICINE, 2022.

## Claims

1. A method for isolating colon epithelial crypts, comprising:
a) providing a dissected colon;
b) opening a dissected colon longitudinally;
c) incubating the colon in a non-enzymatic cell recovery solution;
d) shaking the colon in sucrose solution;
e) subjecting the crypts to centrifugation, and
f) removing supernatant, thereby obtaining isolated epithelial crypts.

2. The method according to claim 1, further comprising, after step c) and before step d), incubating the colon in a chelating solution.

3. The method according to claim 2, wherein the chelating solution is EDTA or EGTA solution.

4. The method according to any one of claims 1 to 3, further comprising:
g) seeding the isolated crypts in the culture medium.

5. The method according to any one of claims 1 to 4, wherein the step e) and/or step g) is performed in the presence of a Rho-kinase inhibitor.

6. The method according to any one of claims 1 to 5, wherein the crypts are obtained from a mammal colon.

7. The method according to claim 6, wherein the crypts are obtained from a rodent colon.

8. The method according to claim 7, wherein the crypts are obtained from a mouse colon.

9. The method according to claim 8, wherein the crypts are obtained from:
a) normal mice without genetic mutation, or
b) transgenic mice with a known genetic mutation.

10. Isolated intestinal epithelial crypts, obtained by the method according to any one of claims 1 to 9.

11. A method for obtaining primary intestinal epithelial cells, comprising:
a) Seeding isolated epithelial crypts according to claim 10 in a culture medium comprising a Rho-kinase inhibitor;
b) Subjecting the culture medium to the conditions suitable for cultivating epithelial cells.

12. A method for preparation of immortalized intestinal epithelial cells from primary intestinal epithelial cells, comprising the steps:
a) Providing primary intestinal epithelial cells;
b) Immortalizing the cells by:
(i) Inactivating the CDKN2A locus, and
(ii) introducing an hTERT transgene.

13. The method according to claim 12, wherein steps (i) and (ii) are performed sequentially or simultaneously.

14. The method according to claim 12 or 13, wherein the primary intestinal epithelial cells are obtained by the method according to claim 11.

15. An immortalized epithelial cell, comprising:
a) an inactivated CDKN2A locus, and
b) expressing hTERT transgene.
